# EUROPEAN PATENT APPLICATION

(11) **EP 4 414 350 A1**
(43) Date of publication of application: **14.08.2024**
(21) Application number: 22878241.3
(22) Date of filing: 01.09.2022
(51) Int. Cl.: C07C 17/358, B01J 23/04, C07B 37/08, C07B 61/00, C07C 21/18

(54) **(E)-1,1,1,4,4,4-HEXAFLUORO-2-BUTENE PRODUCTION METHOD**

(30) Priority: 06.10.2021 JP 2021164799
(71) Applicant: Resonac Corporation, Tokyo 105-7325 (JP)
(72) Inventor: OHTSUKI Kazuaki, Tokyo 105-8518 (JP); KOBAYASHI Hiroshi, Tokyo 105-8518 (JP); TANIMOTO Yosuke, Tokyo 105-8518 (JP)
(74) Representative: Strehl Schübel-Hopf & Partner
(86) International application number: PCT/JP2022/032976
(87) International publication number: WO 2023/058370

(57) **Abstract**

Provided is a method capable of producing (E)-1,1,1,4,4,4-hexafluoro-2-butene at a high yield even with a catalyst containing alumina having a large sodium content. The method for producing (E)-1,1,1,4,4,4-hexafluoro-2-butene includes an isomerization step of bringing (Z)-1,1,1,4,4,4-hexafluoro-2-butene into contact with a catalyst containing alumina to isomerize the (Z)-1,1,1,4,4,4-hexafluoro-2-butene to (E)-1,1,1,4,4,4-hexafluoro-2-butene. The alumina is a porous body having a plurality of pores and having a central pore size of 5 nm or more and 40 nm or less. The proportion of the total volume of pores having a pore size of not less than -50% and not more than +50% of the central pore size, of all the pores of the porous body, to the total pore volume of the porous body is 70% or more.

## Description

### Technical Field

The present invention relates to a method for producing (E)-1,1,1,4,4,4-hexafluoro-2-butene.

### Background Art

1,1,1,4,4,4-Hexafluoro-2-butene is useful as a refrigerant for air conditioners, coolers, heat pumps, and the like, as a working fluid for Rankine cycles, or as a bubble expander.

1,1,1,4,4,4-Hexafluoro-2-butene includes two geometric isomers having different properties: (E)-1,1,1,4,4,4-hexafluoro-2-butene (boiling point: 7.5°C) and (Z)-1,1,1,4,4,4-hexafluoro-2-butene (boiling point: 33.5°C). In the present description, (E)-1,1,1,4,4,4-hexafluoro-2-butene may also be called an "E-isomer", and (Z)-1,1,1,4,4,4-hexafluoro-2-butene may also be called a "Z-isomer".

In the above various applications, the E-isomer may be useful from properties including the boiling point, but synthesizing 1,1,1,4,4,4-hexafluoro-2-butene yields a mixture of the two geometric isomers. There is therefore a demand for the technology of producing the E-isomer at a high yield. For example, PTL 1 discloses a technology of isomerizing the Z-isomer to the E-isomer by bringing the Z-isomer into contact with a catalyst containing alumina.

### Citation List

### Patent Literature

PTL 1: WO 2015/059500

### Summary of Invention

### Technical Problem

The technology disclosed in PTL 1, however, yields the E-isomer at a high yield with a catalyst containing sodium at a low content (350 ppm by mass) but is difficult to yield the E-isomer at a high yield with a catalyst containing sodium at a high content (2,170 ppm by mass).

Sodium has a function of inhibiting the catalytic activity of alumina or a function of inhibiting the reaction that promotes disproportionation to isomerize the Z-isomer to the E-isomer, and thus a catalyst containing sodium at a low content is preferred. Alumina is, however, typically produced by the Bayer process and thus contains 0.2 to 0.7% by mass of sodium in many cases. It is not easy to remove sodium from alumina, and the removal involves complicated operations.

The present invention is intended to provide a production method capable of yielding (E)-1,1,1,4,4,4-hexafluoro-2-butene at a high yield even with a catalyst containing alumina having a large sodium content when (Z)-1,1,1,4,4,4-hexafluoro-2-butene is brought into contact with a catalyst containing alumina and is isomerized to yield (E)-1,1,1,4,4,4-hexafluoro-2-butene.

### Solution to Problem

To solve the problems, aspects of the present invention are the following [1] to [6].
[1] A method for producing (E)-1,1,1,4,4,4-hexafluoro-2-butene, the method including an isomerization step of bringing (Z)-1,1,1,4,4,4-hexafluoro-2-butene into contact with a catalyst containing alumina to isomerize the (Z)-1,1,1,4,4,4-hexafluoro-2-butene to (E)-1,1,1,4,4,4-hexafluoro-2-butene, in which
   the alumina is a porous body having a plurality of pores and having a central pore size of 5 nm or more and 40 nm or less, and
   the proportion of the total volume of pores having a pore size of not less than -50% and not more than +50% of the central pore size, of all the pores of the porous body, to the total pore volume of the porous body is 70% or more.
[2] The method for producing (E)-1,1,1,4,4,4-hexafluoro-2-butene according to the aspect [1], in which the porous body has a total pore volume of 0.5 mL/g or more and 1.6 mL/g or less.
[3] The method for producing (E)-1,1,1,4,4,4-hexafluoro-2-butene according to the aspect [1] or [2], in which the alumina is a hydrogen fluoride-treated material prepared by bringing hydrogen fluoride gas into contact.
[4] The method for producing (E)-1,1,1,4,4,4-hexafluoro-2-butene according to any one of the aspects [1] to [3], in which in the isomerization step, the (Z)-1,1,1,4,4,4-hexafluoro-2-butene is brought into contact with the catalyst under conditions of a temperature of 20°C or more and 400°C or less and a pressure of 3 MPa or less.
[5] The method for producing (E)-1,1,1,4,4,4-hexafluoro-2-butene according to any one of the aspects [1] to [4], in which the catalyst further contains a metal oxide.
[6] The method for producing (E)-1,1,1,4,4,4-hexafluoro-2-butene according to any one of the aspects [1] to [5], in which the catalyst further contains sodium at 3,000 ppm by mass or less.

### Advantageous Effects of Invention

According to the present invention, (E)-1,1,1,4,4,4-hexafluoro-2-butene can be produced at a high yield even with a catalyst containing alumina having a large sodium content when (Z)-1,1,1,4,4,4-hexafluoro-2-butene is brought into contact with a catalyst containing alumina and is isomerized to yield (E)-1,1,1,4,4,4-hexafluoro-2-butene.

### Description of Embodiments

Embodiments of the present invention will now be described. The embodiments are merely examples of the present invention, and the present invention is not limited to the embodiments. Various modifications or improvements can be made in the embodiments, and such modifications and improvements can be encompassed by the present invention.

The method for producing (E)-1,1,1,4,4,4-hexafluoro-2-butene pertaining to the present embodiment includes an isomerization step of bringing (Z)-1,1,1,4,4,4-hexafluoro-2-butene into contact with a catalyst containing alumina (Al₂O₃) to isomerize the (Z)-1,1,1,4,4,4-hexafluoro-2-butene to (E)-1,1,1,4,4,4-hexafluoro-2-butene.

The alumina is a porous body having a plurality of pores and having a central pore size of 5 nm or more and 40 nm or less. The proportion of the total volume of pores having a pore size of not less than -50% and not more than +50% of the central pore size (hereinafter also called the "proportion of pores having a central pore size of ±50%"), of all the pores of the porous body, to the total pore volume of the porous body is 70% or more.

By such a production method, (E)-1,1,1,4,4,4-hexafluoro-2-butene can be produced at a high yield even with a catalyst containing alumina having a large sodium (Na) content.

Only the Z-isomer may be brought into contact or a mixture of the Z-isomer and the E-isomer may be brought into contact with the catalyst. Alternatively, a mixture of the Z-isomer and an additional substance may be brought into contact with the catalyst. Examples of the additional substance include inert gases such as nitrogen gas (N₂) and argon (Ar).

The isomerization of the Z-isomer in the isomerization step may be performed in either the liquid phase or the gas phase. The isomerization of the Z-isomer in the isomerization step may be performed by using a well-known chemical industrial method including a batch process and a continuous process. After the isomerization step, a collection step of separating and collecting the E-isomer from the resulting product may be performed.

Hereinafter, the method for producing (E)-1,1,1,4,4,4-hexafluoro-2-butene pertaining to the present embodiment will be described in further detail.

### [Alumina]

The alumina in the catalyst in the present invention may be a support supporting another material, may be supported on a support of another material, or may be independent particles not in the form of a support. In any of the forms, alumina has the activity as a catalyst.

The pore size, the central pore size, the pore volume (V), and the total pore volume of alumina are determined by using a mercury porosimeter assuming a pore is a cylindrical model. While the pressure is changed when mercury is injected under pressure, pores having a pore size of 3.5 nm or more and 177 µm or less are measured. In the present invention, the "pore size" and the "central pore size" mean the "pore diameter" and the "center pore diameter", respectively. The "total pore volume" is the total volume of pores having a pore size of 3.5 nm or more and 177 µm or less.

In the present invention, the "central pore size" is, on the curved line in which a value (dV/d(log D)) calculated by differentiating a pore volume (V) by the logarithm (log D) of a pore size (D) is plotted against the pore size (D), the pore size at a maximum value when the differentiated value (dV/d(log D)) has a maximum or the pore size at a maximum absolute value when the differentiated value has no maximum,.

To produce the E-isomer at a high yield even with a catalyst containing alumina having a large sodium content, the alumina as a porous body is required to have a central pore size of 5 nm or more and 40 nm or less and preferably has a central pore size of 6 nm or more and 35 nm or less. In the alumina as a porous body, the proportion of pores having a central pore size of ±50% to the total pore volume is required to be 70% or more.

Of such alumina, an alumina in which the central pore size is 25 nm or more and 35 nm or less and the proportion of pores having a central pore size of ±50% to the total pore volume is 70% or more and an alumina in which the central pore size is 6 nm or more and 10 nm or less and the proportion of pores having a central pore size of ±50% to the total pore volume is 70% or more are more preferred. The proportion of pores having a central pore size of ±50% to the total pore volume is particularly preferably 90% or more.

To produce the E-isomer at a high yield even with a catalyst containing alumina having a large sodium content, the alumina as a porous body preferably has a total pore volume of 0.5 mL/g or more and 1.6 mL/g or less and more preferably 0.55 mL/g or more and 1.4 mL/g or less.

The alumina in which the central pore size is 5 nm or more and 40 nm or less and the proportion of pores having a central pore size of ±50% to the total pore volume is 70% or more is available as commercial products. For example, aluminums NST-3 and NST-7 manufactured by NIKKI-UNIVERSAL fall into the alumina satisfying the conditions of central pore size and proportion of pores having a central pore size of ±50%.

The catalyst containing alumina may be used in any appropriate shape known in the art. For example, when the catalyst is used in a fixed bed or a fluidized bed, a pellet or granular catalyst having dimensions suitable for use in the fixed bed or the fluidized bed may be used for ease of handling the catalyst that is packed into or discharged from a reactor.

### [Method for producing alumina]

Alumina may be produced by any method that yields an alumina satisfying the conditions of central pore size and proportion of pores having a central pore size of ±50%, and a sol-gel/oil drop process as an industrial production method can yield an alumina satisfying the above conditions. An example of the method for producing alumina through the sol-gel/oil drop process will next be described.

To an aluminum alkoxide solution diluted with an organic solvent, water diluted with an organic solvent is added for hydrolysis, and an alumina precursor sol is prepared. The alumina precursor sol is then allowed to stand at room temperature to a temperature of 60°C for 1 to 14 days and is gelated into an alumina precursor gel. The alumina precursor gel is then maintained at a temperature not more than the boiling point of the solvent of the aluminum alkoxide solution, and the reduced amount of the solvent in the alumina precursor gel is controlled by temperature conditions or holding time. Accordingly, an alumina gel in which the central pore size is 5 nm or more and 40 nm or less and the proportion of pores having a central pore size of ±50% to the total pore volume is 70% or more is produced.

As an example, a gel containing an alumina having an intended pore size distribution can be prepared by changing the standing time of an alumina precursor sol. For example, when aluminum n-butoxide ((C₄H₉O)₃Al) is used as the aluminum alkoxide, standing the gel at room temperature for 7 days or more yields a gel of an alumina in which the proportion of pores having a central pore size of ±50% to the total pore volume is 70% or more. By increasing the standing time, a gel of an alumina having a smaller pore size distribution can be prepared.

As another example, a gel containing an alumina having an intended central pore size can be prepared by changing the temperature at which an alumina precursor sol is allowed to stand. For example, when aluminum n-butoxide is used as the aluminum alkoxide, standing the gel at room temperature for one or more days yields a gel of an alumina having a central pore size of 5 nm or more and 10 nm or less. Standing the gel at 60°C for one or more days yields a gel of an alumina having a central pore size of 10 nm or more and 30 nm or less. By increasing the standing temperature, a gel of an alumina having a larger central pore size can be prepared.

When the above alumina gel is dried and the dried gel is burnt, an alumina in which the central pore size is 5 nm or more and 40 nm or less and the proportion of pores having a central pore size of ±50% to the total pore volume is 70% or more can be produced. For example, by burning the dried gel in the atmosphere at a temperature of 400°C or more, an alumina in which the central pore size is 5 nm or more and 40 nm or less and the proportion of pores having a central pore size of ±50% to the total pore volume is 70% or more can be produced.

As described above, the sol-gel/oil drop process enables appropriate control of the bulk density, the specific surface area, the total pore volume, the pore size, the pore distribution, or the like of the produced alumina by controlling chemical properties of a spherical sol or by changing chemical operations of sol-gel. Accordingly, the conditions of central pore size and proportion of pores having a central pore size of ±50% can be appropriately controlled.

The alumina satisfying the condition of central pore size and the condition of proportion of pores having a central pore size of ±50% can also be produced by the method as described below. In other words, a trialkylaluminum is decomposed into aluminum hydroxide monohydrate (Al(OH)₃•H₂O), and the aluminum hydroxide monohydrate is burnt into γ-alumina. This enables the production of the alumina satisfying the above conditions.

The alumina produced by the above method from a trialkylaluminum as the starting material is pressed or extruded into pellets, and the resulting alumina has a higher purity than the naturally derived alumina and has a satisfactory pore size distribution with uniformized pore sizes. The alumina produced by the above method from a trialkylaluminum as the starting material is commercially available as an activated alumina used as a catalyst support.

### [Additional component to catalyst]

The catalyst containing alumina may contain an additional component. Examples of the additional component include a metal oxide. When the catalyst containing alumina contains an additional component, the catalyst preferably contains a metal oxide as the additional component from the viewpoint of improving the catalytic activity. A catalyst containing a metal oxide may further contain at least one of a metal (hereinafter called an "additional metal") and a compound of the metal (hereinafter called an "additional metal compound") as the additional component. The type of the metal of the metal oxide may be the same as or different from the type of the metal of the additional metal or the additional metal compound.

The amount of the metal contained as the additional component in the catalyst may be appropriately changed. From the viewpoint of the catalytic activity and the limit of the supported amount on alumina, however, the amount of the metal contained as the additional component is preferably 5% by mass or more and 30% by mass or less of the whole catalyst.

The additional component may be contained in the catalyst while supported on alumina. A catalyst containing alumina that supports a metal oxide can be produced by, for example, impregnating a commercially available sodium-containing alumina with the metal oxide. A catalyst containing alumina that supports a metal oxide and at least one of an additional metal and an additional metal compound can be produced by impregnating a commercially available sodium-containing alumina with the metal oxide and at least one of the additional metal and the additional metal compound.

Impregnating a sodium-containing alumina with a metal oxide, an additional metal, or an additional metal compound can be achieved by any appropriate method known in the catalyst production field. For example, alumina may be impregnated with a metal oxide, an additional metal, or an additional metal compound and then subjected to a thermochemical treatment including heating and burning.

The metals of the metal oxide, the additional metal, and the additional metal compound as the additional components may be any type, and examples include nickel (Ni), cobalt (Co), iron (Fe), manganese (Mn), chromium (Cr), copper (Cu), and silver (Ag). The metal sources of these metals include an inorganic salt, an organic salt, and the like of such a metal.

For example, when the metal species is chromium, examples of the metal source include chromium chloride, chromium nitrate, chromium hydroxide, chromium sulfate, chromium carbonate, basic chromium carbonate, chromium formate, chromium oxalate, chromium acetate, chromium oxide, chromium sesquioxide, chromium dioxide, chromium sesquioxide hydrate, chromium(II) fluoride, chromium(III) fluoride, chromium(II) fluoride hydrate, chromium oxyfluoride, and chromium sulfide. If desired, metallic chromium can be used as the metal source. Of them, chromium chloride, chromium nitrate, chromium sulfate, and the like, which are soluble in water, are specifically preferred.

When the metal species is cobalt, iron, manganese, nickel, copper, silver, or the like, a metal salt can be used as the metal source as with chromium. When the metal species is cobalt, iron, manganese, nickel, copper, silver, or the like, a chloride, a nitrate, a sulfate, and the like are specifically preferred as with chromium.

An example method of impregnating alumina with a metal oxide is the following method: alumina is immersed in an aqueous metal salt solution; the alumina is allowed to absorb the aqueous metal salt solution; and then the whole is dried and is further burnt. In the method, the burnt product may be subjected to hydrogen fluoride treatment of bringing hydrogen fluoride gas (HF) into contact, and the alumina may be converted into a hydrogen fluoride-treated material. Alternatively, the burnt product may be subjected to reduction treatment of bringing hydrogen gas (H₂) into contact to reduce the metal salt into metal, and then the metal may be further subjected to hydrogen fluoride treatment.

In other words, a metal or a compound thereof is preferably supported on alumina as the support, and then the whole is preferably brought into contact with hydrogen fluoride gas in the gas phase for hydrogen fluoride treatment. By the hydrogen fluoride treatment, at least a part of the metal supported on alumina may be reacted with hydrogen fluoride to form a metal fluoride. When a metal fluoride is supported on alumina as the support, the hydrogen fluoride treatment may be unnecessary.

As described above, in the method of impregnating alumina with a metal oxide, alumina is immersed in an aqueous metal salt solution, and then the whole is dried and burnt. The drying is preferably performed at a temperature of 50°C or more and 120°C or less. The burning is preferably performed at a temperature of 200°C or more and 500°C or less while oxygen gas (O₂) or a mixed gas of oxygen gas and an inert gas is allowed to flow. Examples of the inert gas include nitrogen gas and argon, and examples of the mixed gas include air. When burning is performed while air is allowed to flow, the air space velocity SV (converted at 0°C and 1 atmosphere (0.1 MPa)) is preferably 100/h or more and 1,000/h or less.

At a burning temperature of 200°C or more, a catalyst having a sufficient catalytic activity can be produced. At a burning temperature of 500°C or less, the catalyst effective surface area becomes sufficient, and thus a catalyst having a sufficient catalytic activity can be produced.

When an alumina supporting a metal salt is dried and then is burnt while air is allowed to flow, sudden heat generation may be involved at a burning temperature of 150°C or more. Hence, burning is preferably performed while the temperature is controlled such that the temperature of alumina is maintained at 500°C or less.

The catalyst prepared as above may be subjected to treatment of coming into contact with hydrogen fluoride, and the metal oxide and the alumina may be partially fluorinated. The hydrogen fluoride treatment can improve the catalytic activity.

The concentration of hydrogen fluoride in the treatment gas used for the hydrogen fluoride treatment is preferably 10% by volume or more and 100% by volume or less. When the concentration of hydrogen fluoride is less than 100% by volume, hydrogen fluoride is mixed with an inert gas to prepare the treatment gas.

The feed rate of the treatment gas in the hydrogen fluoride treatment is preferably 50/h or more and 600/h or less in terms of space velocity SV (converted at 0°C and 1 atmosphere). The treatment temperature in the hydrogen fluoride treatment is preferably 200°C or more and 450°C or less. The hydrogen fluoride treatment involves heat generation and thus the hydrogen fluoride treatment is preferably performed while the temperature is carefully controlled such that the temperature of alumina is maintained at 450°C or less. Even a catalyst containing no metal oxide may be subjected to the above hydrogen fluoride treatment to improve the catalytic activity.

When a catalyst contains a metal oxide supported on alumina, the amount of the alumina in the whole catalyst is preferably 50% by mass or more, more preferably 60% by mass or more, even more preferably 70% by mass or more, and particularly preferably 80% by mass or more.

When a catalyst contains a metal oxide supported on alumina, the amount of the metal oxide in the whole catalyst is preferably less than 50% by mass, more preferably 40% by mass or less, even more preferably 30% by mass or less, and particularly preferably 20% by mass or less.

### [Sodium content of catalyst]

The catalyst pertaining to the present embodiment may contain any amount of sodium, and the catalyst may have a sodium content of, for example, 800 ppm by mass or more. The sodium content of a catalyst can be determined by any appropriate known method. Examples of the particularly useful method include atomic absorption spectrophotometry (AAS) and emission spectrography (OES) such as inductively coupled plasma emission spectrometry (ICP-OES). In Examples and Comparative Examples described later, inductively coupled plasma emission spectrometry was used to determine the sodium content of a catalyst.

The sodium content of a catalyst can be controlled as follows: alumina is impregnated with an aqueous sodium salt solution, dried, and burnt. Examples of the sodium salt include sodium fluoride (NaF), sodium nitrate (NaNO₃), and sodium chloride (NaCl).

Sodium may be generated during the production of alumina, and thus alumina may originally contain sodium. The catalyst may have a sodium content of 800 ppm by mass or more as described above, and accordingly, when the catalyst contains a metal oxide supported on alumina, the alumina may originally contain sodium at a content of 800 ppm by mass or more. Hence, even an alumina having a high sodium content, for example, an alumina having a sodium content of 800 ppm by mass or more and 3,000 ppm by mass or less, may be used to produce the catalyst pertaining to the present embodiment. In other words, in the present invention, even when a catalyst containing alumina has a sodium content of 800 ppm by mass or more and 3,000 ppm by mass or less, the E-isomer can be produced at a high yield, and such an alumina can be used to produce the catalyst pertaining to the present embodiment.

At least one of the alumina and the metal oxide used in the catalyst pertaining to the present embodiment may be a crystalline material or an amorphous material. For example, when a catalyst is analyzed by X-ray diffraction method, the catalyst may exhibit or may not exhibit crystalline characteristics.

When the catalyst pertaining to the present embodiment is used to isomerize the Z-isomer, the catalytic activity may decrease over time. In such a case, heat treatment in air at a temperature of 200°C or more and 600°C or less can reactivate the catalyst. As the atmosphere for the heat treatment, air as described above as well as an inert gas such as nitrogen gas or hydrogen fluoride gas may be used. As the atmosphere for the hydrogen fluoride treatment of a catalyst, an inert gas such as nitrogen gas or air may be used to reactivate the catalyst.

### [Isomerization step]

The method for producing (E)-1,1,1,4,4,4-hexafluoro-2-butene pertaining to the present embodiment includes an isomerization step of bringing the Z-isomer into contact with a catalyst containing alumina to isomerize the Z-isomer to the E-isomer. The temperature condition or the pressure condition in the isomerization step is not specifically limited. To produce the E-isomer at a high yield even with a catalyst containing alumina having a large sodium content, the temperature is preferably 0°C or more and 400°C or less, more preferably 20°C or more and 400°C or less, even more preferably 120°C or more and 400°C or less, particularly preferably 200°C or more and 360°C or less, and most preferably 240°C or more and 320°C or less.

The pressure may be atmospheric pressure (0.1 MPa), a pressure less than atmospheric pressure, or a pressure more than atmospheric pressure, but is preferably 3 MPa or less, more preferably 0.2 MPa or less, even more preferably 0.1 MPa or more and 0.15 MPa or less, and particularly preferably 0.1 MPa or more and 0.12 MPa or less.

The reaction time in the isomerization step, that is, the preferred contact time between a starting material containing the Z-isomer and a catalyst can vary with the size of a reactor, reaction conditions, or the like. For example, when an Inconel (registered trademark) tube having an outer diameter of 2.7 cm is used as the reactor, 60 mL of a catalyst is packed into the reactor, a Z-isomer previously heated at 80°C is fed thereto, and isomerization is performed, the contact time is preferably 1 second or more and 120 seconds or less, more preferably 1 second or more and 60 seconds or less, and even more preferably 1 second or more and 30 seconds or less. The contact time may be reduced by increasing the flow rate of a starting material fed to a catalyst.

The isomerization step may be performed in the absence of hydrogen fluoride. To prevent or delay excessive decomposition of a starting material containing the Z-isomer or coking of a catalyst, the isomerization step is preferably performed in the presence of a small amount of hydrogen fluoride in some cases. When hydrogen fluoride is used, the molar ratio of hydrogen fluoride to the Z-isomer (hydrogen fluoride:Z-isomer) is preferably 0.01:1 to 1:1, more preferably 0.1:1 to 1:1, and even more preferably 0.5:1 to 1:1.

The isomerization step may be performed in any appropriate production apparatus. The isomerization step may be performed by either method of a batch process or a continuous process.

By either method of a batch process or a continuous process, the isomerization step may be performed in a one-pot manner using a single reaction region or reactor or may be performed by using a plurality of reaction regions or reactors.

For efficient isomerization, a continuous process is preferably used, but the operation of a production apparatus for isomerization is required to be temporarily stopped in some cases for maintenance or reactivation of the catalyst.

The reactor may be any type, and for example, a sealed metal tube containing a catalyst may be used as the reactor. When a starting material containing the Z-isomer is fed to a reactor, the Z-isomer comes into contact with the catalyst and is isomerized to the E-isomer, and a product containing the E-isomer is discharged out of the reactor. The isomerization reaction can be performed mainly in the gas phase.

The reactor is preferably made of a corrosion resistant material. Devices attached to the reactor, such as tubes connected to the reactor, a distillation column used to purify the E-isomer, and tubes connected to the distillation column, are also preferably made of a corrosion resistant material.

Examples of the corrosion resistant material include stainless steel (specifically austenite stainless steel), high nickel alloy, Monel (registered trademark), nickelcopper alloy, Hastelloy (registered trademark), nickel alloy, Inconel (registered trademark), nickel-chromium alloy, and copper-clad steel.

By selecting the catalyst type or selecting reaction conditions including temperature and pressure, the Z-isomer can be isomerized to the E-isomer at a yield of 85% or more. By appropriately performing the selection, the E-isomer can be produced at a yield of 90% or more, 950 or more, or 99% or more. By the above isomerization reaction, the product contains almost no compounds other than the Z-isomer and the E-isomer (i.e., the selectivity to the E-isomer is almost 100%), and thus the conversion rate of the Z-isomer is the yield of the E-isomer.

The product by the isomerization step contains the E-isomer. The content of the E-isomer in the product (the purity of the E-isomer) may vary with the selection of the catalyst type or the selection of reaction conditions including temperature and pressure as described above, but the purity may be high enough not to need further purification. When further purification is needed, a method such as fractional distillation is performed to collect the E-isomer from the product, and a high purity E-isomer can be produced.

The product produced by the isomerization step may contain an unreacted Z-isomer. In such a case, a method such as fractional distillation may be performed to collect the unreacted Z-isomer from the product. The collected Z-isomer may be subjected to the isomerization step once again as the starting material.

### Examples

The present invention will next be described more specifically with reference to examples and comparative examples.

### [Example 1]

A spherical activated alumina produced by the sol-gel/oil drop process was prepared. The activated alumina was a porous body having a plurality of pores and having a central pore size of 30 nm. The proportion of the total volume of pores having a pore size of 15 nm or more and 45 nm or less (i.e., pores having a pore size of not less than -50% and not more than +50% of the central pore size), of all the pores of the porous body, to the total pore volume of the porous body was 85%. The activated alumina has a total pore volume of 1.3 mL/g.

In 30 mL of pure water, 25 mg of sodium fluoride was dissolved to prepare an aqueous sodium fluoride solution. In the aqueous sodium fluoride solution, 25 g of the activated alumina was immersed to allow the activated alumina to absorb the whole amount of the aqueous sodium fluoride solution. Next, the wet activated alumina having absorbed the aqueous sodium fluoride solution was dried on a hot water at a temperature of 90°C and then was dried for another 3 hours in an air-circulation type hot-air drier controlled at a temperature of 70°C. This yielded an activated alumina having an alumina content of about 99% by mass and a sodium content of 1,000 ppm by mass.

The activated alumina prepared as above was packed into a reactor. The reactor was an Inconel (registered trademark) tube having an outer diameter of 2.7 cm and an inner diameter of 2.5 cm. While nitrogen gas was allowed to flow through the reactor at a flow rate of 200 mL/min, the temperature in the reactor (i.e., the temperature of the activated alumina) was controlled at 340°C, and the activated alumina was heated in the nitrogen gas stream. The activated alumina was heated for 10 hours.

Next, the flow rate of nitrogen gas was increased to 675 mL/min, and hydrogen fluoride gas was fed at a flow rate of 75 mL/min into the reactor. During the feeding, the temperature in the reactor was maintained at 340°C. Accordingly, the activated alumina was subjected to a treatment of bringing hydrogen fluoride gas into contact and was made into a hydrogen fluoride-treated material, and a catalyst was prepared. After nitrogen gas and hydrogen fluoride gas were fed for 14 hours, the hydrogen fluoride gas feeding was stopped, and the temperature in the reactor was reduced to 280°C. Nitrogen purge in the reactor was then performed.

To the reactor after the nitrogen purge, (Z)-1,1,1,4,4,4-hexafluoro-2-butene previously heated at 80°C was fed at a flow rate of 100 mL/min. Accordingly, the Z-isomer was brought into contact with the catalyst to be isomerized. As for the isomerization conditions, the temperature was 280°C, the pressure was 0.10 MPa, and the reaction time was 0.6 min. The produced gas discharged from the reactor was cooled to -78°C to yield (E)-1,1,1,4,4,4-hexafluoro-2-butene.

A part of the produced gas discharged from the reactor was sampled and was analyzed by using a gas chromatograph-mass spectrometer, and compounds contained in the produced gas were identified and quantified. The result revealed that isomerization of the Z-isomer yielded the E-isomer at a yield of 99% by mole. The results are illustrated in Table 1.

**[Table 1]**

| | Central pore size (nm) | Proportion of pores having central pore size of ±50% (%) | Total pore volume (mL/g) | Sodium content (ppm by mass) | Yield (%) |
|---|---|---|---|---|---|
| Ex. 1 | 30 | 85 | 1.3 | 1000 | 99 |
| Ex. 2 | 30 | 85 | 1.3 | 20 | 99 |
| Ex. 3 | 30 | 85 | 1.3 | 2000 | 99 |
| Ex. 4 | 30 | 85 | 1.3 | 3000 | 96 |
| Ex. 5 | 30 | 71 | 1.3 | 1000 | 90 |
| Ex. 6 | 30 | 71 | 1.3 | 20 | 98 |
| Ex. 7 | 30 | 71 | 1.3 | 2000 | 89 |
| Ex. 8 | 30 | 71 | 1.3 | 3000 | 85 |
| Comp. Ex. 1 | 30 | 62 | 1.5 | 1000 | 63 |
| Comp. Ex. 2 | 30 | 62 | 1.5 | 20 | 90 |
| Comp. Ex. 3 | 30 | 62 | 1.5 | 2000 | 56 |
| Comp. Ex. 4 | 30 | 62 | 1.5 | 3000 | 45 |
| Ex. 9 | 8 | 94 | 0.6 | 1000 | 99 |
| Ex. 10 | 8 | 94 | 0.6 | 20 | 99 |
| Ex. 11 | 8 | 94 | 0.6 | 2000 | 98 |
| Ex. 12 | 8 | 94 | 0.6 | 3000 | 95 |
| Ex. 13 | 8 | 72 | 0.6 | 1000 | 99 |
| Ex. 14 | 8 | 72 | 0.6 | 20 | 99 |
| Ex. 15 | 8 | 72 | 0.6 | 2000 | 96 |
| Ex. 16 | 8 | 72 | 0.6 | 3000 | 90 |
| Comp. Ex. 5 | 8 | 61 | 0.4 | 1000 | 71 |
| Comp. Ex. 6 | 8 | 61 | 0.4 | 20 | 92 |
| Comp. Ex. 7 | 8 | 61 | 0.4 | 2000 | 66 |
| Comp. Ex. 8 | 8 | 61 | 0.4 | 3000 | 51 |
| Comp. Ex. 9 | 58 | 89 | 5.0 | 1000 | 81 |
| Comp. Ex. 10 | 58 | 89 | 5.0 | 20 | 90 |
| Comp. Ex. 11 | 58 | 89 | 5.0 | 2000 | 80 |
| Comp. Ex. 12 | 58 | 89 | 5.0 | 3000 | 76 |
| Comp. Ex. 13 | 58 | 72 | 5.4 | 1000 | 71 |
| Comp. Ex. 14 | 58 | 72 | 5.4 | 20 | 89 |
| Comp. Ex. 15 | 58 | 72 | 5.4 | 2000 | 68 |
| Comp. Ex. 16 | 58 | 72 | 5.4 | 3000 | 61 |
| Comp. Ex. 17 | 4 | 82 | 0.4 | 1000 | 86 |
| Comp. Ex. 18 | 4 | 82 | 0.4 | 20 | 96 |
| Comp. Ex. 19 | 4 | 82 | 0.4 | 2000 | 80 |
| Comp. Ex. 20 | 4 | 82 | 0.4 | 3000 | 75 |
| Comp. Ex. 21 | 4 | 71 | 0.4 | 1000 | 80 |
| Comp. Ex. 22 | 4 | 71 | 0.4 | 20 | 96 |
| Comp. Ex. 23 | 4 | 71 | 0.4 | 2000 | 78 |
| Comp. Ex. 24 | 4 | 71 | 0.4 | 3000 | 70 |

The analyzer used for gas chromatography and analysis conditions were as follows:
Apparatus: gas chromatograph GC-2014s manufactured by Shimadzu Corporation
Column: CarboPak B 60/80 SP-1000
Column temperature: 150°C (5 min) - temperature rise at 10°C/min (5 min) - 200°C (20 min)
Injection temperature: 200°C
Carrier gas: helium (He)
Detector: hydrogen flame ionization detector (FID)

The central pore size, the proportion of pores having a central pore size of ±50%, and the total pore volume of the activated alumina were determined by using a mercury porosimeter, Auto Pore IV 9520 manufactured by Micromeritics. Specifically, 0.2 g of the activated alumina was placed in a dedicated cell having a volume of 2 mL, and the dedicated cell was installed in the mercury porosimeter. The mercury porosimeter was then operated to inject mercury into the activated alumina at a pressure of 1.5 kPa to 350 MPa.

In the mercury intrusion method, when a pore in alumina is assumed to be a cylindrical model, the relationship between the pore size D of the first cylindrical pore into which mercury intrudes and the pressure P at the intrusion satisfies D = -4σcosθ/P (where σ is the surface tension of mercury (480 mN/m); and θ is the contact angle (130°)). In other words, a smaller pore size requires a higher pressure, and the pore size D is determined from the pressure P in accordance with the above equation. The mercury intrusion volume V at the pressure P is the pore volume (V) of the corresponding pore size D. Hence, by determining the mercury intrusion volume V at any pressure P while the pressure is changed, the mercury intrusion volume V of the corresponding pore size D can be determined.

The central pore size was the pore size D at which the mercury intrusion volume V increased the most, that is, the pore size D at the maximum value (maximum peak) of dV/d(log D) on the curved line in which a value dV/d(log D) calculated by differentiating a mercury intrusion volume V by the logarithm log D of a pore size D was plotted against the pore size D.

The sodium content of a catalyst was determined by inductively coupled plasma emission spectrometry (ICP-OES). The used analyzer was an ICP emission spectrophotometer, ICPE-9000 manufactured by Shimadzu Corporation. Specifically, a solution in which 0.1 g of a catalyst was dissolved in an aqueous nitric acid solution at a concentration of 5% by mass was used as the sample, and the sodium content was analyzed.

### [Examples 2 to 4]

The Z-isomer was isomerized to the E-isomer in the same manner as in Example 1 except that a catalyst was prepared such that the sodium content of the catalyst was the numerical value illustrated in Table 1 by changing the amount of sodium fluoride. The results are illustrated in Table 1.

### [Example 5]

The Z-isomer was isomerized to the E-isomer in the same manner as in Example 1 except that an activated alumina in which the central pore size was 30 nm, the proportion of pores having a central pore size of ±50% (pores having a pore size of 15 nm or more and 45 nm or less) to the total pore volume was 71%, and the total pore volume was 1.3 mL/g was used. The activated alumina used here was a spherical activated alumina produced by the sol-gel/oil drop process but differed from that used in Example 1, and the central pore size, the proportion of pores having a central pore size of ±50%, and the total pore volume were determined in the same manner as in Example 1. The results are illustrated in Table 1.

### [Examples 6 to 8]

The Z-isomer was isomerized to the E-isomer in the same manner as in Example 5 except that a catalyst was prepared such that the sodium content of the catalyst was the numerical value illustrated in Table 1 by changing the amount of sodium fluoride. The results are illustrated in Table 1.

### [Example 9]

The Z-isomer was isomerized to the E-isomer in the same manner as in Example 1 except that an activated alumina in which the central pore size was 8 nm, the proportion of pores having a central pore size of ±50% (pores having a pore size of 4 nm or more and 12 nm or less) to the total pore volume was 94%, and the total pore volume was 0.6 mL/g was used. The activated alumina used here was a spherical activated alumina produced by the sol-gel/oil drop process but differed from that used in Example 1 or Example 5, and the central pore size, the proportion of pores having a central pore size of ±50%, and the total pore volume were determined in the same manner as in Example 1. The results are illustrated in Table 1.

### [Examples 10 to 12]

The Z-isomer was isomerized to the E-isomer in the same manner as in Example 9 except that a catalyst was prepared such that the sodium content of the catalyst was the numerical value illustrated in Table 1 by changing the amount of sodium fluoride. The results are illustrated in Table 1.

### [Example 13]

The Z-isomer was isomerized to the E-isomer in the same manner as in Example 1 except that an activated alumina in which the central pore size was 8 nm, the proportion of pores having a central pore size of ±50% (pores having a pore size of 4 nm or more and 12 nm or less) to the total pore volume was 72%, and the total pore volume was 0.6 mL/g was used. The activated alumina used here was a spherical activated alumina produced by the sol-gel/oil drop process but differed from that used in Example 1, Example 5, or Example 9, and the central pore size, the proportion of pores having a central pore size of ±50%, and the total pore volume were determined in the same manner as in Example 1. The results are illustrated in Table 1.

### [Examples 14 to 16]

The Z-isomer was isomerized to the E-isomer in the same manner as in Example 13 except that a catalyst was prepared such that the sodium content of the catalyst was the numerical value illustrated in Table 1 by changing the amount of sodium fluoride. The results are illustrated in Table 1.

### [Examples 17 to 22]

The Z-isomer was isomerized to the E-isomer in the same manner as in Example 1 except that the temperature and the pressure in the isomerization reaction were changed as illustrated in Table 2. The results are illustrated in Table 2.

**[Table 2]**

| | Central pore size (nm) | Proportion of pores having central pore size of ±50% (%) | Total pore volume (mL/g) | Sodium content (ppm by mass) | Isomerization reaction | | Yield (%) |
|---|---|---|---|---|---|---|---|
| | | | | | Temperature (°C) | Pressure (MPa) | |
| Ex. 17 | 30 | 85 | 1.3 | 1000 | 120 | 0.10 | 90 |
| Ex. 18 | 30 | 85 | 1.3 | 1000 | 240 | 0.10 | 98 |
| Ex. 19 | 30 | 85 | 1.3 | 1000 | 360 | 0.10 | 95 |
| Ex. 20 | 30 | 85 | 1.3 | 1000 | 400 | 0.10 | 89 |
| Ex. 21 | 30 | 85 | 1.3 | 1000 | 280 | 0.15 | 99 |
| Ex. 22 | 30 | 85 | 1.3 | 1000 | 280 | 0.34 | 97 |

### [Examples 23 to 26]

The Z-isomer was isomerized to the E-isomer in the same manner as in Example 1 except that the step of giving an activated alumina was changed to the step described below and the isomerization reaction was performed at a temperature as illustrated in Table 2. In Example 26, the sodium content of the catalyst was also changed. The results are illustrated in Table 2.

In other words, 5 g of chromium chloride and 25 mg of sodium fluoride were dissolved in 30 mL of pure water to prepare an aqueous solution containing chromium chloride and sodium fluoride. In the aqueous sodium fluoride solution, 25 g of activated alumina was immersed to allow the activated alumina to absorb the whole amount of the aqueous sodium fluoride solution. Next, the wet activated alumina having absorbed the aqueous sodium fluoride solution was dried on a hot water at a temperature of 90°C and then was dried for another 3 hours in an air-circulation type hot-air drier controlled at a temperature of 70°C. The catalyst was burnt in an air stream at 400°C for 3 hours to yield an activated alumina having an alumina content of about 80% by mass, a chromia content of about 20% by mass, and a sodium content of 1,000 ppm by mass.

**[Table 3]**

| | Central pore size (nm) | Proportion of pores having central pore size of ±50% (%) | Total pore volume (mL/g) | Sodium content (ppm by mass) | Isomerization reaction | | Yield (%) |
|---|---|---|---|---|---|---|---|
| | | | | | Temperature (°C) | Pressure (MPa) | |
| Ex. 23 | 30 | 85 | 1.3 | 1000 | 120 | 0.10 | 93 |
| Ex. 24 | 30 | 85 | 1.3 | 1000 | 240 | 0.10 | 99 |
| Ex. 25 | 30 | 85 | 1.3 | 1000 | 280 | 0.10 | 99 |
| Ex. 26 | 30 | 85 | 1.3 | 20 | 280 | 0.10 | 99 |
| Ex. 27 | 30 | 85 | 1.3 | 1000 | 280 | 0.10 | 95 |

### [Example 27]

The Z-isomer was isomerized to the E-isomer in the same manner as in Example 1 except that no hydrogen fluoride treatment was performed in the catalyst preparation. The results are illustrated in Table 3. The results illustrated in Table 3 revealed that a catalyst containing a metal oxide had excellent catalytic activity.

### [Comparative Example 1]

The Z-isomer was isomerized to the E-isomer in the same manner as in Example 1 except that an activated alumina in which the central pore size was 30 nm, the proportion of pores having a central pore size of ±50% (pores having a pore size of 15 nm or more and 45 nm or less) to the total pore volume was 620, and the total pore volume was 1.5 mL/g was used. The activated alumina used here was a spherical activated alumina produced by the sol-gel/oil drop process but differed from that used in Examples, and the central pore size, the proportion of pores having a central pore size of ±50%, and the total pore volume were determined in the same manner as in Example 1. The results are illustrated in Table 1.

### [Comparative Examples 2 to 4]

The Z-isomer was isomerized to the E-isomer in the same manner as in Comparative Example 1 except a catalyst was prepared such that the sodium content of the catalyst was the numerical value illustrated in Table 1 by changing the amount of sodium fluoride. The results are illustrated in Table 1.

### [Comparative Example 5]

The Z-isomer was isomerized to the E-isomer in the same manner as in Example 1 except that the central pore size was 8 nm, the proportion of pores having a central pore size of ±50% (pores having a pore size of 4 nm or more and 12 nm or less) to the total pore volume was 610, and the total pore volume was 0.4 mL/g. The activated alumina used here was a spherical activated alumina produced by the sol-gel/oil drop process but differed from that used in Examples or Comparative Example 1, and the central pore size, the proportion of pores having a central pore size of ±50%, and the total pore volume were determined in the same manner as in Example 1. The results are illustrated in Table 1.

### [Comparative Examples 6 to 8]

The Z-isomer was isomerized to the E-isomer in the same manner as in Comparative Example 5 except that a catalyst was prepared such that the sodium content of the catalyst was the numerical value illustrated in Table 1 by changing the amount of sodium fluoride. The results are illustrated in Table 1.

### [Comparative Example 9]

The Z-isomer was isomerized to the E-isomer in the same manner as in Example 1 except that an activated alumina in which the central pore size was 58 nm, the proportion of pores having a central pore size of ±50% (pores having a pore size of 29 nm or more and 87 nm or less) to the total pore volume was 89%, and the total pore volume was 5.0 mL/g was used. The activated alumina used here was a spherical activated alumina produced by the sol-gel/oil drop process but differed from that used in Examples, Comparative Example 1, or Comparative Example 5, and the central pore size, the proportion of pores having a central pore size of ±50%, and the total pore volume were determined in the same manner as in Example 1. The results are illustrated in Table 1.

### [Comparative Examples 10 to 12]

The Z-isomer was isomerized to the E-isomer in the same manner as in Comparative Example 9 except that a catalyst was prepared such that the sodium content of the catalyst was the numerical value illustrated in Table 1 by changing the amount of sodium fluoride. The results are illustrated in Table 1.

### [Comparative Example 13]

The Z-isomer was isomerized to the E-isomer in the same manner as in Example 1 except that an activated alumina in which the central pore size was 58 nm, the proportion of pores having a central pore size of ±50% (pores having a pore size of 29 nm or more and 87 nm or less) to the total pore volume was 72%, and the total pore volume was 5.4 mL/g was used. The activated alumina used here was a spherical activated alumina produced by the sol-gel/oil drop process but differed from that used in Examples, Comparative Example 1, Comparative Example 5, or Comparative Example 9, and the central pore size, the proportion of pores having a central pore size of ±50%, and the total pore volume were determined in the same manner as in Example 1. The results are illustrated in Table 1.

### [Comparative Examples 14 to 16]

The Z-isomer was isomerized to the E-isomer in the same manner as in Comparative Example 13 except that a catalyst was prepared such that the sodium content of the catalyst was the numerical value illustrated in Table 1 by changing the amount of sodium fluoride. The results are illustrated in Table 1.

### [Comparative Example 17]

The Z-isomer was isomerized to the E-isomer in the same manner as in Example 1 except that an activated alumina in which the central pore size was 4 nm, the proportion of pores having a central pore size of ±50% (pores having a pore size of 2 nm or more and 6 nm or less) to the total pore volume was 82%, and the total pore volume was 0.4 mL/g was used. The activated alumina used here was a spherical activated alumina produced by the sol-gel/oil drop process but differed from that used in Examples, Comparative Example 1, Comparative Example 5, Comparative Example 9, or Comparative Example 13, and the central pore size, the proportion of pores having a central pore size of ±50%, and the total pore volume were determined in the same manner as in Example 1. The results are illustrated in Table 1.

### [Comparative Examples 18 to 20]

The Z-isomer was isomerized to the E-isomer in the same manner as in Comparative Example 17 except that a catalyst was prepared such that the sodium content of the catalyst was the numerical value illustrated in Table 1 by changing the amount of sodium fluoride. The results are illustrated in Table 1.

### [Comparative Example 21]

The Z-isomer was isomerized to the E-isomer in the same manner as in Example 1 except that an activated alumina in which the central pore size was 4 nm, the proportion of pores having a central pore size of ±50% (pores having a pore size of 2 nm or more and 6 nm or less) to the total pore volume was 71%, and the total pore volume was 0.4 mL/g was used. The activated alumina used here was a spherical activated alumina produced by the sol-gel/oil drop process but differed from that used in Examples, Comparative Example 1, Comparative Example 5, Comparative Example 9, Comparative Example 13, or Comparative Example 17, and the central pore size, the proportion of pores having a central pore size of ±50%, and the total pore volume were determined in the same manner as in Example 1. The results are illustrated in Table 1.

### [Comparative Examples 22 to 24]

The Z-isomer was isomerized to the E-isomer in the same manner as in Comparative Example 21 except that a catalyst was prepared such that the sodium content of the catalyst was the numerical value illustrated in Table 1 by changing the amount of sodium fluoride. The results are illustrated in Table 1.

As revealed from the results illustrated in Table 1, Examples 1 to 16 satisfied the conditions that the central pore size was 5 nm or more and 40 nm or less and the proportion of pores having a central pore size of ±50% to the total pore volume was 70% or more. Accordingly, in Examples 1 to 16, the E-isomer was produced at a high yield not only with a catalyst having a small sodium content of 20 ppm by mass but also with a catalyst having a high sodium content of 1,000 ppm by mass to 3,000 ppm by mass.

In contrast, in Comparative Examples 1 to 8, a condition of a central pore size of 5 nm or more and 40 nm or less was satisfied, but a condition that the proportion of pores having a central pore size of ±50% to the total pore volume was 70% or more was not satisfied, and thus the E-isomer was produced at an insufficient yield with a catalyst having a sodium content of 1,000 ppm by mass or more.

In Comparative Examples 9 to 24, a condition of a central pore size of 5 nm or more and 40 nm or less was not satisfied, and thus the E-isomer was produced at an insufficient yield with a catalyst having a sodium content of 1,000 ppm by mass or more.

## Claims

1. A method for producing (E)-1,1,1,4,4,4-hexafluoro-2-butene, the method comprising:
an isomerization step of bringing (Z)-1,1,1,4,4,4-hexafluoro-2-butene into contact with a catalyst containing alumina to isomerize the (Z)-1,1,1,4,4,4-hexafluoro-2-butene to (E)-1,1,1,4,4,4-hexafluoro-2-butene, wherein
the alumina is a porous body having a plurality of pores and having a central pore size of 5 nm or more and 40 nm or less, and
a proportion of a total volume of pores having a pore size of not less than -50% and not more than +50% of the central pore size, of all the pores of the porous body, to a total pore volume of the porous body is 70% or more.

2. The method for producing (E)-1,1,1,4,4,4-hexafluoro-2-butene according to claim 1, wherein the porous body has a total pore volume of 0.5 mL/g or more and 1.6 mL/g or less.

3. The method for producing (E)-1,1,1,4,4,4-hexafluoro-2-butene according to claim 1 or claim 2, wherein the alumina is a hydrogen fluoride-treated material prepared by bringing hydrogen fluoride gas into contact.

4. The method for producing (E)-1,1,1,4,4,4-hexafluoro-2-butene according to any one of claims 1 to 3, wherein in the isomerization step, the (Z)-1,1,1,4,4,4-hexafluoro-2-butene is brought into contact with the catalyst under a condition of a temperature of 20°C or more and 400°C or less and a pressure of 3 MPa or less.

5. The method for producing (E)-1,1,1,4,4,4-hexafluoro-2-butene according to any one of claims 1 to 4, wherein the catalyst further contains a metal oxide.

6. The method for producing (E)-1,1,1,4,4,4-hexafluoro-2-butene according to any one of claims 1 to 5, wherein the catalyst further contains sodium at 3,000 ppm by mass or less.
